# EUROPÄISCHE PATENTSCHRIFT

(11) **EP 0 649 827 B1**
(45) Veröffentlichungstag und Bekanntmachung des Hinweises auf die Patenterteilung: **21.01.1998**
(21) Anmeldenummer: 94116048.3
(22) Anmeldetag: 12.10.1994
(51) Int. Cl.: C07C 4/22, C08J 11/12

(54) **Verfahren zur Rückgewinnung von Styrol aus gebrauchtem Polystyrol**
Process for the recovery of styrene from used polystyrene
Procédé pour le récupération de styrène à partir de polystyrène usagé

(30) Priorität: 21.10.1993 DE 4335972
(43) Veröffentlichungstag der Anmeldung: 26.04.1995
(73) Patentinhaber: BASF Aktiengesellschaft, 67063 Ludwigshafen (DE)
(72) Erfinder: Northemann, Andreas, Dr., D-67071 Ludwigshafen (DE)

(56) Entgegenhaltungen:
- EP-A- 0 502 618
- EP-A- 0 577 279
- WO-A-92/04423
- US-A- 3 901 951
- DATABASE WPI Derwent Publications Ltd., London, GB; AN 84-197547[32] & JP-A-59 111 815 (FUJI STAND RES) 28. Juni 1984

## Beschreibung

Der thermische Abbau von Polystyrol zum Monostyrol (Depolymerisation) ist allgemein bekannt und mehrfach beschrieben, z.B. von A. Guyot in Polym. Degrad. Stabil. 15(3), 1986, S. 219-235 oder von H. Ohtani et al. in Eur. Polym. J. 26(8), 1990, S. 893-899.

Nach DE-A-3 037 829 können modifizierte Peche und niedrig siedende Aromaten durch thermische Behandlung von Kunststoffabfällen erhalten werden, die polymeres Styrol enthalten. Dies geschieht in Gegenwart hochsiedender Aromaten, wobei der Abfall dem flüssigen hochsiedenden Aromaten zugesetzt und gleichzeitig die entstehenden niedersiedenden Aromaten abgetrennt werden.

Aus JA-A-74 05 183 ist es bekannt, 37,5 Teile Polystyrol in 62,5 Teilen Lösungsmittel aufzulösen und bei 600°C zersetzend zu destillieren. Bei dieser Verfahrensweise erhält man Styrol in ca. 80 % Ausbeute bezogen auf eingesetztes Polystyrol.

Eine schonende Möglichkeit des Aufheizens von solchen Polystyrol-Lösungen besteht in der Zufuhr von Wasserdampf;

SU-A-771 079 beschreibt ein Verfahren, bei dem das Polystyrol in einem organischen Lösungsmittel im Verhältnis 1:6 bis 1:10 aufgelöst und anschließend in Gegenwart von Wasserdampf thermisch abgebaut wird. Der Dampf wird im Verhältnis 1:1 zum Polystyrol zugesetzt. Man erhält dabei ca. 77 % Styrol, wobei ca. 80 % des Ausgangsmaterials umgesetzt werden.

Auch SU-A-1 035 017 beschreibt ein Verfahren zur Depolymerisation von Polystyrol in Gegenwart von Wasserdampf. Hierbei wird ein Katalysator, nämlich Eisen-Chrom-Kalium-Oxid verwendet. Das Polymermaterial wird aufgelöst und zusammen mit Wasserdampf (3:1 im Verhältnis zum Polymer) über den Katalysator geleitet. Das Verhältnis Lösungsmittel zu Polymer beträgt ca. 1:1. Die Ausbeute an Monomer soll ca. 85 % bezogen auf eingesetztes Polymermaterial erreichen.

JA-A-2 029 492 beschreibt ein Verfahren, bei dem Polystyrol in flüssiger Form thermisch abgebaut wird und die entweichenden, niedrigsiedenden Bestandteile in der Gasphase an einem Zeolith zu Kohlenwasserstoffen und aromatischen Verbindungen kondensiert werden.

GB-A-2 228 493 beschreibt ein Verfahren zur Wiedergewinnung von Styrol durch pyrolytische Reduktion von Polystyrolabfall. Bei diesem gemischt katalytisch-thermischen Flüssig- und Gasphasenverfahren erhält an Styrol zu etwa 70-75 % und benötigt eine Blei-Zink-Legierung als Wärmeüberträger.

WO-A-92 04423, EP-A-0 502 618 und US-A-3 901 951 beschreiben Verfahren zur Pyrolyse von Kunststoffabfällen allgemein in der Wirbelschicht, bei denen als Wärmeträger unspezifizierte Materialien, u.a. Sand, Kohlenstoff, Aluminiumoxid, amorphes Aluminiumsilikat, unbestimmte "Katalysatoren" und keramisches Material ("ceramics") vorgeschlagen werden.

Diese Vorschläge können jedoch jedenfalls für die Pyrolyse von Polystyrol nicht als unmittelbar technisch umsetzbar angesehen werden: Erstens weisen sie keine Wärmeträger aus, die die erforderliche chemische Indifferenz besitzen. Es hat sich nämlich gezeigt, daß es, abweichend von den - wenn auch undifferenzierten - Vorschlägen, "Katalysatoren" zu verwenden, speziell bei der Pyrolyse von Polystyrol darauf ankommt, gerade keine katalytischen Effekte hervorzurufen, sondern eine rein thermische Spaltung zu bewirken, um die - allerdings bekannte - Neigung von Polystyrol zur thermischen Depolymerisation auszunützen.

Außerdem sind keine Wärmeträger in Betracht gezogen worden, die chemische Indifferenz mit der erforderlichen Langlebigkeit (Abriebfestigkeit) verbinden.

Schließlich gelingt es nach den Angaben in der WO-A-92 04423 schon deshalb nicht, eine hohe Rückgewinnungsrate für monomeres Styrol zu erreichen, weil die dort angegebenen Versuche bei einer hierfür viel zu hohen Temperatur ausgeführt wurden, sodaß auch Spaltprodukte von Styrolmonomer wie Ethylen und Toluol in großer Menge gebildet wurden.

Auch alle sonst bekannten Verfahren haben Nachteile: Manchmal ist es nötig, das gebrauchte Polystyrol vor der Depolymerisation aufzulösen. Die großen Mengen an Lösungsmittel machen ein solches Verfahren unrentabel, da das Lösungsmittel wiedergewonnen werden muß. Andere Verfahren sind aufgegliedert in zwei Prozeßschritte, das thermische Abbauen und das katalytische Cracken der Abbauprodukte. Darüber hinaus werden zum Teil Katalysatoren eingesetzt, die schnell desaktivieren, so daß bei der Depolymerisation noch zusätzlich Wasserdampf benötigt wird, um dem entgegenzuwirken.

Eine Aufgabe der Erfindung ist es, ein Verfahren anzugeben, das die vorgenannten Nachteile nicht aufweist.

Der Erfindungsgegenstand betrifft ein Verfahren zur Wiedergewinnung von monomerem Styrol aus Styrolpolymere enthaltendem Kunststoffabfall ("Müll") durch thermische Depolymerisation, wobei der Müll flüssig oder fest in eine auf 400-700°C aufgeheizte Wirbelschicht eines festen Wärmeüberträgers gefördert, mit einer mittleren Verweilzeit von weniger als 60 Sekunden gecrackt bzw. depolymerisiert und Styrol aus den gasförmigen Crackprodukten gewonnen wird.

Als Wärmeüberträger wird erfindungsgemäß Magnesium-Aluminium-Silikat, ein unter Reaktionsbedingungen inertes temperaturbeständiges Material verwendet, das vorteilhaft eine Korngröße von 30-500 µm, vorzugsweise zwischen 70 und 350 µm aufweist. Magnesium-Aluminium-Silikat wird auch als Steatit bezeichnet und wird z.B. als Träger für Katalysatoren für die Herstellung von Phthalsäureanhydrid bekannt ist (vgl. Ullmann's Enzyklopedia of Ind. Chem. Vol A. 20, p. 181-185).

Die Depolymerisation von Polystyrol findet bei dem erfindungsgemäßen verfahren rein thermisch statt, so daß kein Katalysator für die Reaktion notwendig ist.

Ein geeignetes Material weist einen Wirbelpunkt im Bereich vcn 0,02-0,07 m/s, vorzugsweise im Bereich von 0,03-0,06 m/s auf. Zur Prüfung, ob es sich wirklich um ein inertes Material handelt, kann man reines Styrol durch die Wirbelschicht fördern. Das Styrol sollte in wenigstens 99 %iger Ausbeute wiedergewonnen werden.

Zur Fluidisation der Wirbelschicht wird ein unter den Reaktionsbedingungen inertes Gas verwendet. Dies können Wasserstoff, Stickstoff, ein Edelgas, Kohlendioxid, aber auch Wasserdampf oder bei der Reaktion entstehende gasförmige Verbindungen sein. Vorzugsweise nimmt man die bei der Reaktion entstehenden gasförmigen Verbindungen, die in diesem Fall im Kreis geführt werden.

Der Müll wird in der bevorzugten Ausführungsform flüssig, evtl. aber auch fest in die Wirbelschicht dosiert. Fördert man festes Polystyrol in die Wirbelschicht, so muß die Verweilzeit deutlich verlängert werden. Bevorzugt schmilzt man das Polystyrol auf und pumpt die Schmelze in den Reaktor. Geschäumtes Polystyrol wird zunächst in einer entsprechenden Mühle entgast. Das Polystyrol kann ohne vorheriges Shreddern oder Zerkleinern aufgeschmolzen werden. Zum Aufschmelzen und Dosieren des Polystyrols eignet sich z.B. ein Extruder, aber auch jede andere Art des Aufschmelzens und Förderns des Polystyrols ist geeignet.

Es ist ein besonderer Vorteil des Verfahrens, daß das Polystyrol unverdünnt als Schmelze in den Reaktor gefördert wird und daß kein weiterer Apparat zum Auflösen verwendet werden muß. Darüberhinaus erlaubt diese Vorgehensweise eine höhere Raumzeitausbeute als die bekannten Verfahren.

Die Reaktorinnentemperatur sollte zwischen 400-700°C liegen. Höhere Temperatur ist zwar denkbar, führt jedoch verstärkt zur Bildung niedermolekularer gasförmiger Substanzen. Bei niedrigerer Temperatur nimmt die Geschwindigkeit der Depolymerisationsreaktion stark ab; das Verfahren ist dann i.a. weniger wirtschaftlich. Besonders bevorzugt ist ein Temperaturbereich zwischen 450 und 650°C.

Die Depolymerisation des Polystyrols wird vorzugsweise bei atmosphärischem Druck ausgeführt, kann aber auch bei einem Druck ober- oder unterhalb Atmosphärendruck ablaufen.

Die Verweilzeit im Reaktor wird durch den Wirbelgasstrom einreguliert. Sie liegt im allgemeinen im Bereich unterhalb von 60 Sekunden, z.B. zwischen 0,5 und 40 Sekunden; bevorzugt ist ein Bereich zwischen 2 und 25 Sekunden. Die Verweilzeit wird durch die Größe des verwendeten Reaktors und durch den Wirbelpunkt des im Reaktor befindlichen Wirbelmaterials mitbestimmt. Die Verweilzeit spielt insbesondere bei der Art der Dosierung eine wichtige Rolle. Beim Dosieren von festem Polystyrol liegt die Verweilzeit z.B. im Bereich um 30 Sekunden, während die Verweilzeit bei Flüssigdosierung eher bei 4-10 Sekunden liegt.

In der bevorzugten Ausführungsform wird der den Wirbelschichtreaktor verlassende Gasstrom zweistufig kondensiert, um eine Vortrennung bestimmter Siedefraktionen zu erreichen. Ein erster Kondensator wird bei einer Temperatur oberhalb des Siedepunktes von Styrol, vorzugsweise bei 160-200°C betrieben. Ein nachgeschalteter zweiter Kondensator wird bei erheblich niedrigerer Temperatur, vorzugsweise bei bis zu 40°C betrieben, um die bei dieser Temperatur flüssigen Bestandteile abzuscheiden.

Die in diesem niedrigeren Temperaturbereich weiter gasförmigen Bestandteile werden zur Fluidisation des Wirbelbettes mit Hilfe eines Verdichters im Kreis geführt. Nur ein kleiner Überschußanteil wird ausgeschleust und etwa zur Energiegewinnung verwertet. Die im ersten Kondensator zwischen dem Siedepunkt des Styrols und 200°C abgeschiedene Fraktion wird wieder in den Prozeß zurückgeführt. Darüber hinaus dient dieser Teil dazu, die für den Gesamtprozeß benötigte Energie bereitzustellen, durch Verbrennung eines Teils dieser Fraktion. Die für den Prozeß benötigte Energie hängt stark vom Raum-Zeit-Verhalten der Anlage ab. Bei der bevorzugten Durchführungsform beträgt der Anteil an Kondensat, der zur Dekkung des Energiebedarfs herangezogen wird, 50-100 % der zwischen dem Siedepunkt des Styrols und 200°C abgeschiedenen Fraktion. Aus der Fraktion, die zwischen 100 und 170°C siedet und im wesentlichen das monomere Styrol enthält, kann das Styrol durch Destillation gewonnen und wiederverwertet werden.

In der bevorzugten Ausführungsform dieses Verfahrens werden alle anfallenden Fraktionen und Stoffströme genutzt. Durch das teilweise energetische Verwerten von Fraktionen, die kein monomeres Styrol enthalten, kann das Verfahren energieautark, also ohne Verbindung zu bestehenden Chemieanlagen und damit wirtschaftlich betrieben werden. Dieses Verfahren leistet somit einen wertvollen Beitrag zum rohstofflichen Recycling von Polystyrolabfall.

### Beispiel 1

Zur Veranschaulichung im Labormaßstab werden in einen Wirbelschichtreaktor von 7 cm Innendurchmesser und 30 cm Höhe aus Stahl 1340 g oder 0,93 Liter Mg-Al-Silikat mit einer Korngröße von 150-250 µm und einem Wirbelpunkt von 0,047 m/sec gefüllt und über einen innen liegenden Wärmetauscher auf 550°C aufgeheizt. Zuvor aufgeschmolzener Polystyrolmüll, der einen Gehalt von 90 % Styrol, berechnet als Monomer aufweist, wird mit einer Rate von 0,2 kg/h in den Reaktor gepumpt. Zur Fluidisation der Wirbelschicht werden stündlich 140 Normalliter Gas in den Reaktor eingeblasen, wobei sich eine Verweilzeit von 4 Sekunden einstellt. Der den Reaktor verlassende Gasstrom wird zweistufig kondensiert. Der erste Kondensator wird zur Abscheidung von höhersiedenden Komponenten bei einer Temperatur von 170°C, der zweite Kondensator wird zur Abscheidung des Styrols bei einer Temperatur von 20°C betrieben. Mittels eines Verdichters wird der nicht kondensierbare Anteil zur Fluidisation der Wirbelschicht verwendet. Über einen Zeitraum von 10 Stunden wurden die beiden kondensierten Fraktionen gesammelt und anschließend analysiert.

In dem bei 170°C betriebenen Kondensator werden 187 g (9,4 % bzgl. Einsatz) einer Fraktion abgeschieden, die aus einer nicht näher erfaßten Mischung aus Dimerstyrol, Trimerstyrol und höher siedenden wachsartigen Substanzen besteht. In dem bei 20°C betriebenen Kondensator werden 1720 g (86 % bzgl. Einsatz) abgeschieden, die aus 87 Gew.-% Styrol, 5 Gew.-% Ethylbenzol und Toluol, 5 Gew.-% methylierten Styrolderivaten und 3 Gew.-% nicht näher identifizierter Aromaten besteht. Das entspricht einer Ausbeute an Styrol (bezogen auf eingesetztes Polymer) von 84 %.

Die Gasfraktion, die 5,6 Gew.-% vom Einsatz ausmacht und im wesentlichen aus C₁-C₆-Kohlenwasserstoffen besteht, wird zur Fluidisation der Wirbelschicht im Kreis geführt.

### Beispiele 2-6

Man verfährt entsprechend den allgemeinen Angaben des Beispiels 1, wobei Temperatur und Verweilzeit systematisch variiert werden. Die nachstehende Tabelle zeigt die Ergebnisse.

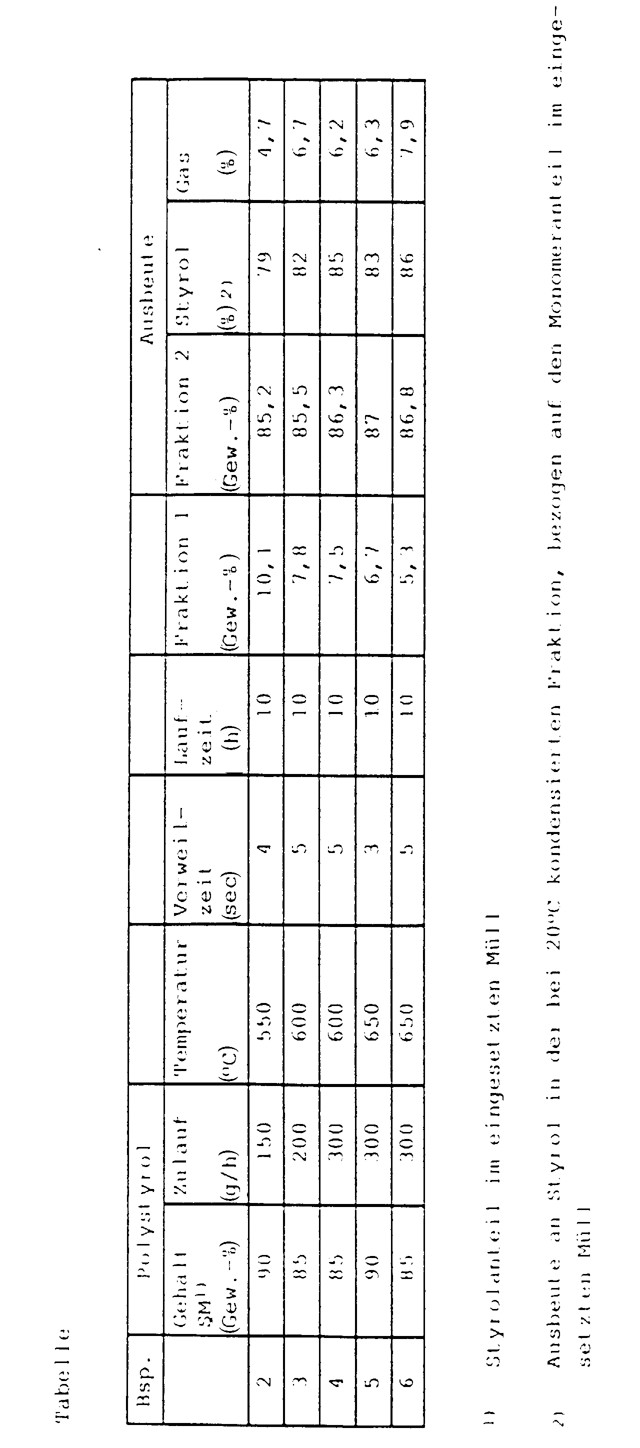

### Beispiel 7

Es wurde unter den Bedingungen des Beispiels 6 verfahren und die Produktivität des Verfahrens über einen längeren Zeitraum ermittelt. Die Laufzeit des Versuchs betrug 1000 h. Während dieser Zeit wurden 30 kg Polystyrolabfall depolymerisiert. Dabei wurden aus dem Abfall, der zu 90 % aus einpolymerisiertem Styrol bestand, 23,2 kg Styrol (Ausbeute 86 %) erhalten. Die bei 170°C kondensierte Fraktion, die 5,1 Gew.-% der eingesetzten Polymermenge ausmachte und einen Heizwert von 41 MJ/kg aufwies, wurden zur Energieerzeugung genutzt, die Gasfraktion wurde zur Fluidisation der Wirbelschicht im Kreis geführt.

## Patentansprüche

1. Verfahren zur Wiedergewinnung von monomerem Styrol aus Styrolpolymere enthaltendem Kunststoffabfall durch thermische Depolymerisation, wobei der Kunststoffabfall flüssig oder fest in eine auf 400-700°C aufgeheizte Wirtelschicht eines festen Wärmeüberträgers gefördert, mit einer mittleren Verweilzeit von weniger als 60 Sekunden gecrackt bzw. depolymerisiert und Styrol aus den gasförmigen Crackprodukten gewonnen wird, dadurch gekennzeichnet, daß als Wärmeüberträger Magnesium-Aluminium-Silikat verwendet wird.

2. Verfahren nach Anspruch 1, dadurch gekennzeichnet, daß als Betriebsgas zur Aufrechterhaltung der Wirbelschicht bei der Depolymerisation als Nebenprodukt gebildete C₁- bis C₆-Kohlenwasserstoffe im Kreis geführt werden.

3. Verfahren nach Anspruch 1, dadurch gekennzeichnet, daß die Kondensation der Crackprodukte in zwei oder mehr Stufen vorgenommen wird, wobei eine erste Kondensationsstufe bei einer Temperatur von 160 bis 200°C und eine zweite Stufe von bis zu 40°C vorgesehen ist und wobei Styrol aus der zweiten Kondensationsstufe gewonnen wird.

4. Verfahren nach Anspruch 1, gekennzeichnet durch einen Wirbelpunkt im Bereich von 0,02 bis 0,07 m/s bzw. eine Teilchengröße von 30 bis 500 µm.

## Claims

1. A process for recovering monomeric styrene from plastics waste containing styrene polymers by thermal depolymerization, in which the plastics waste in liquid or solid form is transported into a fluidized bed of a solid heat transfer agent heated to 400-700°C and is cracked or depolymerized in an average residence time of less than 60 seconds and styrene is obtained from the gaseous crack products, wherein the heat transfer agent used is magnesium aluminum silicate.

2. A process as claimed in claim 1, wherein C₁-C₆-hydrocarbons formed as byproducts in the depolymerization are circulated as operating gas for maintaining the fluidized bed.

3. A process as claimed in claim 1, wherein the condensation of the crack products is carried out in two or more stages, a first condensation stage at from 160 to 200°C and a second stage at up to 40°C being provided and styrene being obtained from the second condensation stage.

4. A process as claimed in claim 1, comprising a fluidization point of 0.02-0.07 m/s or a particle size of 30-500 µm.

## Revendications

1. Procédé pour la récupération de styrène monomère à partir de déchets de matières synthétiques contenant des polymères de styrène, par dépolymérisation thermique, dans lequel on fait passer les déchets de matières synthétiques sous forme liquide ou solide à travers un lit fluidisé chauffé à une température de 400-700°C d'un échangeur de chaleur solide, on les soumet à un craquage, respectivement à une dépolymérisation avec un temps de séjour moyen inférieur à 60 secondes et on récupère le styrène des produits de craquage gazeux, caractérisé en ce qu'on utilise comme échangeur de chaleur du silicate de magnésium-aluminium.

2. Procédé selon la revendication 1, caractérisé en ce qu'on recycle, à titre de gaz produits dans l'usine pour le maintien du lit fluidisé lors de la dépolymérisation, des hydrocarbures en C₁-C₆ obtenus sous forme de produits secondaires.

3. Procédé selon la revendication 1, caractérisé en ce qu'on procède à la condensation des produits de craquage en deux étapes ou plus, dans laquelle on prévoit une première étape de condensation à une température de 160 à 200°C et une seconde étape à une température allant jusqu'à 40°C, et dans laquelle on récupère le styrène de la seconde étape de condensation.

4. Procédé selon la revendication 1, caractérisé par un point de fluidisation dans le domaine de 0,02 à 0,07 m/s, respectivement une granulométrie de 30 à 500 µm.
